# EUROPÄISCHE PATENTSCHRIFT

(11) **EP 3 030 250 B1**
(45) Veröffentlichungstag und Bekanntmachung des Hinweises auf die Patenterteilung: **28.08.2019**
(21) Anmeldenummer: 14741803.2
(22) Anmeldetag: 07.07.2014
(51) Int. Cl.: A61K 38/04, A61P 19/02

(54) **INTRAARTIKULÄRE APPLIKATION VON PEPSTATIN BEI ARTHROSE**
INTRAARTICULAR APPLICATION OF PEPSTATIN IN THE CASE OF ARTHROSIS
APPLICATION INTRA-ARTICULAIRE DE PEPSTATINE DANS LE CAS DE L'ARTHROSE

(30) Priorität: 06.08.2013 EP 13003923
(43) Veröffentlichungstag der Anmeldung: 15.06.2016
(73) Patentinhaber: Merck Patent GmbH, 64293 Darmstadt (DE)
(72) Erfinder: KLEIN, Markus, 64291 Darmstadt (DE); WODOPIA, Ralf, 69469 Weinheim (DE); GUEHRING, Hans, 65343 Eltville (DE); LINDEMANN, Sven, 64291 Darmstadt (DE)
(86) Internationale Anmeldenummer: PCT/EP2014/001861
(87) Internationale Veröffentlichungsnummer: WO 2015/018472

(56) Entgegenhaltungen:
- WO-A1-2012/107153
- JP-A- 2000 300 266
- US-A- 3 906 085
- DATABASE BIOSIS [Online] BIOSCIENCES INFORMATION SERVICE, PHILADELPHIA, PA, US; RUECHEL R ET AL: "MODULATION OF EXPERIMENTAL SYSTEMIC MURINE CANDIDOSIS BY INTRAVENOUS PEPSTATIN", XP002137713, gefunden im BIOSIS Database accession no. PREV199191001749

## Beschreibung

Die vorliegende Erfindung betrifft pharmazeutische Zubereitungen und Arzneimitel zur intraartikulären Applikation, enthaltend Pepstatin sowie deren Herstellung und insbesondere deren Verwendung bei der Behandlung und/oder Prophylaxe von Arthrose, traumatischen Knorpelverletzungen, Arthritis, Schmerz, Allodynie oder Hyperalgesie, besonders bevorzugt bei Arthrose.

### Hintergrund der Erfindung

Arthrose ist die weltweit am weitesten verbreitete Gelenkserkrankung und radiologische Hinweise auf Arthrose findet man in der Mehrheit der über 65-Jährigen. Trotz dieser wesentlichen Bedeutung für das Gesundheitssystem bleiben die Ursachen der Arthrose bislang unklar und wirksame vorbeugende Maßnahmen weiterhin ein entferntes Ziel. Die Verringerung des Gelenkspaltes (bedingt durch die Zerstörung des Gelenkknorpels), verbunden mit Veränderungen im subchondralen Knochen und Osteophytenbildung sind die radiologischen Charakteristiken der Erkrankung. Für den Patienten stehen jedoch Schmerzen (belastungsabhängige und nächtliche Ruheschmerzen) mit nachfolgenden Funktionseinschränkungen im Vordergrund. Diese sind es auch, die den Patienten in die soziale Isolierung mit entsprechenden Folgeerkrankungen treiben.

Der Begriff Arthrose bezeichnet nach einer nicht-amtlichen Definition in Deutschland einen "Gelenkverschleiß", der das altersübliche Maß übersteigt. Ursächlich werden ein Übermaß an Belastung (etwa erhöhtes Körpergewicht), angeborene oder traumatisch bedingte Ursachen, wie Fehlstellungen der Gelenke oder auch knöcherne Deformierungen durch Knochenerkrankungen wie Osteoporose gesehen. Die Arthrose kann ebenfalls als Folge einer anderen Erkrankung, beispielsweise einer Gelenkentzündung (Arthritis) entstehen (sekundäre Arthrose) oder mit überlastungsbedingter Ergussbildung (sekundäre Entzündungsreaktion) einhergehen (aktivierte Arthrose). Die anglo-amerikanische Fachliteratur unterscheidet zwischen der Osteoarthrose (engl. osteoarthritis [OA]), bei welcher die Zerstörung der Gelenkflächen wahrscheinlich hauptsächlich auf Belastungseinwirkungen zurückzuführen sind, und der Arthritis (engl. arthritis, rheumatoid arthritis [RA]), bei welcher die Gelenkdegeneration durch eine Entzündungskomponente im Vordergrund steht.

Grundsätzlich unterscheidet man die Arthrose auch nach ihrer Ursache. Der Arthrosis alcaptonurica liegt eine vermehrte Ablagerung von Homogenitinsäure in Gelenken bei vorbestehender Alkaptonurie zugrunde. Bei der hämophilen Arthrose liegen regelmäßige intraartikuläre Blutungen bei Hämophilie (Blutergelenk) vor. Die Arthrosis urica wird durch den mechanischen Einfluss von Uratkristallen (Harnsäure) auf den gesunden Knorpel hervorgerufen (W. Pschyrembel et al.: Klinisches Wörterbuch mit klinischen Syndromen und einem Anhang Nomina Anatomica. Verlag Walter de Gruyter & Co, 253. Auflage, 1977).

Klassische Ursache einer Arthrose stellt die Dysplasie von Gelenken dar. Am Beispiel der Hüfte wird deutlich, dass die mechanisch am meisten belastete Zone bei einer physiologischen Hüftstellung eine deutlich größere Fläche darstellt, als bei einer dysplastischen Hüfte. Die Belastungen durch die auf das Gelenk einwirkenden Kräfte sind von der Gelenkform jedoch weitgehend unabhängig. Sie verteilen sich im Wesentlichen auf die Hauptbelastungszone(n). Dadurch wird bei einer kleineren Zone eine höhere Druckbelastung als bei einer größeren auftreten. Die biomechanische Druckbelastung des Gelenkknorpels ist somit bei einer dysplastischen Hüfte größer als bei physiologischer Hüftstellung. Diese Gesetzmäßigkeit wird allgemein ursächlich für das gehäufte Auftreten arthrotischer Veränderungen an von der anatomischen Idealform abweichenden, tragenden Gelenken gesehen.

Sind die Folgen einer Verletzung für einen vorzeitigen Verschleiß verantwortlich, so spricht man von einer posttraumatischen Arthrose. Als weitere Ursachen einer sekundären Arthrose werden mechanische, entzündliche, metabolische, chemische (Chinolone), trophische, hormonelle, neurologische und genetische Gründe diskutiert. In den meisten Fällen wird als Diagnose jedoch eine idiopatische Arthrose angegeben, womit der Arzt ein scheinbares Fehlen einer ursächlichen Erkrankung zum Ausdruck bringt (H. I. Roach und S. Tilley, Bone and Osteoarthritis F. Bronner und M. C. Farach-Carson (Editors), Verlag Springer, Band 4, 2007).

Medikamentöse Ursachen für eine Arthrose können beispielsweise Antibiotika vom Typ der Gyrasehemmer (Fluorchinolone, wie Ciprofloxacin, Levofloxacin) sein. Diese Arzneimittel führen in schlecht vaskularisierten Geweben (hyaliner Gelenkknorpel, Sehnengewebe) zu einer Komplexierung von Magnesium-Ionen, was zur Folge hat, dass irreversible Schäden am Bindegewebe entstehen. Diese Schäden sind bei Kindern und Jugendlichen in der Regel in der Wachstumsphase ausgeprägter. Tendopathien und Arthropathien sind bekannte Nebenwirkungen dieser Medikamentenklasse. Beim Erwachsenen führen diese Antibiotika nach Informationen von unabhängigen Pharmakologen und Rheumatologen zu einem beschleunigten physiologischen Abbau des hyalinen Gelenkknorpels (M. Menschik et al., Antimicrob. Agents Chemother. 41, 1997, S. 2562-2565; M. Egerbacher et al., Arch. Toxicol. 73, 2000, S. 557-563; H. Chang et al., Scand. J. Infect. Dis. 28, 1996, S. 641-643; A. Chaslerie et al., Therapie 47, 1992, S. 80). Auch eine langjährige Behandlung mit Phenprocoumon kann durch Abnahme der Knochendichte bei Belastungen der Gelenkbinnenstruktur eine Arthrose begünstigen.

Neben dem Alter sind mechanische Überbelastungen, (Mikro-) Traumata, durch Verlust der Sicherungsmechanismen verursachte Destabilisierungen der Gelenke, sowie genetische Faktoren als Risikofaktoren für Osteoarthrose bekannt. Jedoch sind weder die Entstehung noch Interventionsmöglichkeiten vollständig geklärt (H. I. Roach und S. Tilley, Bone and Osteoarthritis F. Bronner und M. C. Farach-Carson (Editors), Verlag Springer, Band 4, 2007).

In einem von Arthrose betroffenen Gelenk ist der Gehalt von Stickstoffmonoxid zeitweise erhöht. Ähnliches konnte durch hohe mechanische Reizung von Knorpelgewebe beobachtet werden (P. Das et al., Journal of Orthopaedic Research 15, 1997, S. 87-93. A. J. Farrell et al. Annals of the Rheumatic Diseases 51, 1992, S. 1219-1222; B. Fermor et al., Journal of Orthopaedic Research 19, 2001, S. 729-737), wohingegen eine mäßige mechanische Stimulation sich eher positiv auswirkt. Damit sind mechanische Krafteinwirkungen ursächlich an dem Voranschreiten der Osteoarthrose beteiligt (X. Liu et al., Biorheology 43, 2006, S. 183-190).

Grundsätzlich verfolgt die Therapie der Arthrose zwei Ziele. Zum einen die Schmerzfreiheit unter üblicher Belastung und zum anderen die Verhinderung mechanischer Einschränkungen oder Veränderungen eines Gelenkes. Diese Ziele können langfristig nicht durch eine Schmerzbehandlung als rein symptomatischer Therapieansatz erreicht werden, da durch diese das Voranschreiten der Erkrankung nicht aufgehalten werden kann. Soll letzteres erreicht werden, muss die Knorpelzerstörung gestoppt werden. Da sich der Gelenksknorpel beim erwachsenen Patienten nicht regenerieren kann, ist die Beseitigung pathogenetischer Faktoren wie Gelenkdysplasien oder Fehlstellungen, die zu vermehrter punktueller Druckbelastung des Gelenkknorpels führen, zusätzlich enorm wichtig.

Schließlich versucht man mit medikamentöser Hilfe die Degenerationsprozesse im Knorpelgewebe zu verhindern oder zu stoppen. Wesentlich für den Funktionszustand des Gelenkknorpels und damit für dessen Widerstandsfähigkeit gegenüber Belastungen ist die extrazelluäre Matrix, die in erster Line aus Kollagenen, Proteoglykanen und Wasser besteht. Zu den Enzymen, die an der Degradation der extrazellulären Matrix beteiligt sind, zählen vor allem die Metalloproteasen, Aggrecanasen und die Cathepsin-Enzyme. Aber auch weitere Enzyme können prinzipiell Knorpelmatrix abbauen, beispielhaft werden Plasmin, Kallikrein, Neutrophilelastase, Tryptase und Chymase genannt.

Cathepsine gehören der Papain-Superfamilie der lysosomalen Proteasen an. Cathepsine sind an der normalem Proteolyse und dem Umsatz von Zielproteinen und Geweben beteiligt, sowie an der Initiierung von proteolytischen Kaskaden und Proenmzyaktivierungen. Darüber hinaus sind sie an der MHC Klasse II Expression beteiligt (Baldwin (1993) Proc. Natl. Acad. Sci., 90: 6796-6800; Mixuochi (1994) Immunol. Lett., 43: 189-193). Eine abnorme Cathepsin-Expression kann allerdings zu schwerwiegenden Erkrankungen führen. So konnte eine erhöhte Cathepsin-Expression in Krebszellen, beispielsweise bei Brust-, Lungen-, Prostata-, Glioblastom-, und Kopf- und Halskrebs nachgewiesen werden und es konnte gezeigt werden, dass Cathepsine mit einem ungenügenden Therapieerfolg bei Brust-, Lungen-, Kopf- und Halskrebs, sowie Gehirntumoren assoziiert sind (Kos et al. (1998) Oncol. Rep., 5: 1349-1361; Yan et al. (1998) Biol. Chem., 379: 113-123; Mort et al. ; (1997) Int. J. Biochem. Cell Biol., 29: 715-720; Friedrick et al. (1999) Eur. J Cancer, 35: 138-144). Außerdem ist augenscheinlich eine abnorme Cathepsin-Expression an der Ausbildung von entzündlichen und nicht-entzündlichen Erkrankungen beteiligt, wie beispielsweise rheumatoider Arthritis und Osteoarthrose (Keyszer (1995) Arthritis Rheum., 38: 976-984).

Der molekulare Mechanismus der Cathepsinaktivität ist nicht vollständig aufgeklärt. Auf der einen Seite wurde gefunden, dass beispielweise eine induzierte Cathepsin-Expression B-Zellen, denen Serum entzogen wird, vor der Apoptose bewahrt und dass eine Behandlung der Zellen mit Antisense-Oligonukleotiden von Cathepsin B eine Apoptose induziert (Shibata et al. (1998) Biochem. Biophys. Res. Commun., 251: 199-20; Isahara et at. (1999) Neuroscience, 91: 233-249). Diese Berichte legen eine anti-apoptotische Rolle der Cathepsine nahe. Diese stehen jedoch im genauen Gegensatz zu früheren Berichten, die Cathepsine als Apoptosemediatoren beschreiben (Roberts et al (1997) Gastroenterology, 113: 1714-1726; Jones et al. (1998) Am. J. Physiol., 275: G723-730).

Cathepsine werden als inaktive Zymogene an Ribosomen synthetisiert und ins lysosomale System überführt. Nach proteolytischer Abspaltung des N-terminalen Propeptids steigt die Cathepsinkonzentration im sauren Milieu der Lysosomen auf bis zu 1 mM an und die Cathepsine werden von den Lysosomen ins extrazelluläre Medium freigesetzt.

Bei den Cathepsinen unterscheidet man die Cysteincathepsine B, C, H, F, K, L, O, S, V und W, die Aspartylcathepsine D und E und das Serincathepsin G.

Beispiele für Cathepsin-Inhibitoren in der klinischen Entwicklung sind Cathepsin K Inhibitoren zur Behandlung von Arthrose und Cathepsin S Inhibitoren zur Behandlung von Arthritis, neuropathischem Schmerz und Psoriasis.

Zu den Aspartylproteasen zählt man neben Cathepsin D auch die HIV Aspartylprotease (HIV-1 Protease), Renin, Pepsin A and C, BACE (Asp2, Memapsin), Plasmepsine und die Aspartylhämoglobinasen (Takahashi, T. et al., Ed. Aspartic Proteinases Structure, Function, Biology and Biomedical Implications (Plenum Press, New York, 1995), Adams, J. et al., Ann. Rep. Med. Chem. 31, 279-288, 1996; Edmunds J. et al., Ann. Rep. Med. Chem. 31, 51-60, 1996; Miller, D. K. et al., Ann. Rep. Med. Chem 31, 249-268, 1996). Cathepsin D ist normalerweise an der Degradation von intrazellulären oder phagozytierten Proteinen beteiligt und spielt somit eine wichtige Rolle im Proteinmetabolismus (Helseth, et al., Proc. Natl. Acad. Sci. USA 81, 3302-3306, 1984), beim Proteinkatabolismus (Kay, et al., Intracellular Protein Catabolism (eds. Katunuma, et al., 155-162, 1989) und bei der Antigenprozessierung (Guagliardi, et al., Nature, 343, 133-139, 1990; Van Noort, et al., J. Biol. Chem., 264, 14159-14164, 1989).

Erhöhte Cathepsin D-Spiegel werden mit einer Reihe von Krankheiten in Zusammenhang gebracht. So korrelieren erhöhte Cathepsin D-Spiegel mit schlechter Prognose bei Brustkrebs und mit erhöhter Zellinvasion und erhöhtem Risiko von Metastasen, sowie kürzerer rezidivfreien Überlebenszeit nach Therapie und einer insgesamt niedrigeren Überlebensrate (Westley B. R. et al., Eur. J. Cancer 32, 15-24, 1996; Rochefort, H., Semin. Cancer Biol. 1:153, 1990; Tandon, A. K. et al., N. Engl. J. Med. 322, 297, 1990). Die Cathepsin D-Sekretionsrate bei Brustkrebs wird durch eine Überexpression des Gens und durch ein verändertes Processing des Proteins vermittelt. Erhöhte Spiegel von Cathepsin D und anderer Proteasen, wie beispielsweise Kollagenase, hergestellt in unmittelbarer Nähe zu einem wachsenden Tumor, könnten dabei die extrazelluläre Matrix in der Tumorumgebung degradieren und hierdurch die Ablösung von Tumorzellen und die Invasion in neue Gewebe über das Lymph- und Kreislaufsystem vermitteln (Liotta L. A., Scientific American Feb:54, 1992; Liotta L. A. and Stetler-Stevenson W. G., Cancer Biol. 1:99, 1990; Liaudet E., Cell Growth Differ. 6:1045-1052, 1995; Ross J. S., Am. J. Clin. Pathol. 104:36-41, 1995; Dickinson A. J., J. Urol. 154:237-241, 1995).

Cathepsin D wird außerdem mit degenerativen Veränderungen im Gehirn in Verbindung gebracht, wie beispielsweise Alzheimer Krankheit. So ist Catepsin D mit der Spaltung des Amyloid-β-Protein-Vorläufers bzw. eines mutanten Vorläufers assoziiert, der die Expression des Amyloid-Proteins in transfizierten Zellen erhöht (Cataldo, A. M. et al., Proc. Natl. Acad. Sci. 87: 3861, 1990; Ladror, U. S. et al., J. Biol. Chem. 269: 18422, 1994, Evin G., Biochemistry 34: 14185-14192, 1995). Das Amyloid-β-Protein, das durch die Proteolyse des Amyloid-β-Protein-Vorläufers entsteht, führt zur Bildung von Plaques im Gehirn und scheint für die Ausbildung der Alzheimer-Krankheit verantwortlich zu sein. Erhöhte Cathepsin D-Spiegel wurden auch in der Zerebrospinalflüssigkeit von Alzheimer-Patienten gefunden und es konnte eine hohe proteolytische Aktivität von Cathepsin D gegenüber dem mutanten Amyloid-β-Protein-Vorläufer gezeigt werden (Schwager, A. L., et al. J. Neurochem. 64:443, 1995). Darüber hinaus wird eine signifikante Zunahme der Cathepsin D-Aktivität in Biopsien von Chorea Huntington-Patienten gemessen (Mantle D., J. Neurol. Sci. 131: 65-70, 1995).

Bei der Ausprägung einer Arthrose spielt Cathepsin D vermutlich auf verschiedenen Ebenen eine wesentliche Rolle. So werden in Hunden mit spontaner Arthrose im Vergleich gesunden Hunden im Gelenkknorpel des Hüftgelenkkopfes erhöhte mRNA-Spiegel von Cathepsin D gemessen (Clements D. N. et al., Arthritis Res. Ther.. 2006; 8(6): R158; Ritchlin C. et al., Scand. J. Immunnol. 40: 292-298, 1994). Auch Devauchelle V. et al. (Genes Immun. 2004, 5(8): 597-608) zeigen bei menschlichen Patienten unterschiedliche Expressionsraten von Cathepsin D bei Arthrose im Vergleich zu rheumatoider Arthritis (siehe auch Keyszer G. M., Arthritis Rheum. 38: 976-984, 1995). Auch bei Mucolipidose scheint Cathepsin D eine Rolle zu spielen (Kopitz J., Biochem. J. 295, 2: 577-580, 1993).

Die lysosomale Endopeptidase Cathepsin D ist die am weitesten verbreitete Proteinase in den Chondrozyten (Ruiz-Romero C. et al., Proteomics. 2005, 5(12): 3048-59). Die proteolytische Aktivität von Cathepsin D ist zudem in kultiviertem Synovium aus Osteoarthrose-Patienten nachgewiesen worden (Bo G. P. et al., Clin. Rheumatol. 2009, 28(2): 191-9) und auch in Synovectomiegewebe von Patienten mit rheumatoider Arthritis findet man eine erhöhte proteolytische Aktivität (Taubert H. et al., Autoimmunity. 2002, 35(3): 221-4). Lorenz et al. (Proteomics. 2003, 3(6): 991-1002) schreiben so auch, dass zwar die lysosomale und sekretierte Aspartylprotease Cathepsin D im Gegensatz zu den Cathepsinen B und L noch nicht bezüglich Arthritis und Arthrose im Detail studiert wurde, jedoch fanden Lorenz et al. höhere Proteinspiegel von Cathepsin D im Synovialgewebe von Patienten mit Arthrose im Vergleich zu Patienten mit rheumatoider Arthritis.

Gedikoglu et al. (Ann. Rheum. Dis. 1986, 45(4): 289-92) konnten ebenfalls eine erhöhte proteolytische Aktivität von Cathepsin D in Synovialgewebe und Byliss und Ali (Biochem. J. 1978, 171(1): 149-54) im Knorpel von Patienten mit Arthrose nachweisen.

Bei Arthrose kommt es zu einem lokalen Absinken des pH-Werts in Bereichen des Knorpels. Dieses Absinken des pH-Werts ist für das Verständnis der katabolen Prozesse im Knorpel von entscheidender Bedeutung.

Bei Arthrose findet man so auch eine direkte Korrelation von niedrigem pH-Wert im Gelenkgewebe und der Schwere und dem Fortschreiten der Erkrankung. Bei einen pH-Wert von 5,5 kommt es zu einem Selbstverdau des Knorpels. Dieser kann in Explantkulturen (beispielsweise von Maus, Rind oder Mensch) fast vollständig durch Pepstatin oder Ritonavir gehemmt werden. Dies legt eine wesentliche Rolle, wenn nicht sogar eine Schlüsselrolle von Cathepsin D bei der Arthrose nahe, da Pepstatin Aspartylproteasen mit einer Ausnahme - BACE1 - hemmt und nur diese beiden Aspartylproteasen im Knorpelgewebe bisher identifiziert wurden. So beschreiben auch Bo G. P. et al. (Clin. Rheumatol. 2009, 28(2): 191-9) die wichtige Rolle von Cathepsin D bei pathologischen Veränderungen in Gelenken.

Der bekannteste Aspartylproteaseinhibitor ist Pepstatin, ein Peptid das ursprünglich aus einer Streptomyces-Kultur isoliert wurde. Pepstatin ist wirksam gegenüber Pepsin, Cathepsin und Renin. Viele Aspartylproteaseinhibitoren wurden deshalb dem Vorbild der Struktur des Pepstatins nachempfunden (U.S. Pat. No. 4,746,648; Umezawa, H., et al., J. Antibiot (Tokyo) 23: 259-62, 1970; Morishima, H., et al., J. Antibiot. (Tokyo) 23: 263-5, 1970; Lin, T. and Williams, H. R., J. Biol. Chem. 254: 11875-83, 1979; Jupp, R. A., et al., Biochem. J. 265: 871-8, 1990; Agarwal, N. S. and Rich, D. H., J. Med. Chem. 29: 2519-24, 1986; Baldwin, E. T., et al., Proc. Natl. Acad. Sci., USA 90: 6796-800, 1993; Francis, S. E. et al., EMBO J 13: 306-17, 1994).

Aspartylproteasen bzw. Cathepsin D werden häufig als Zielproteine für Wirkstoffe zur Behandlung von neurodegenerativen Erkrankungen, kognitiven Störungen, Demenz, Alzheimer, Krebs, Malaria, HIV-Infektion und Erkrankungen des Herzkreislauf- und Gefäßsystems beschrieben und Inhibitoren von Aspartylproteasen oder Cathepsin D werden zur Behandlung dieser Erkrankungen offenbart, wie beispielsweise in der WO 2009013293, EP 1987834, EP 1872780, EP 1867329, EP 1745778, EP 1745777, EP 1745776, WO 1999002153, WO 1999055687, US 6150416, WO 2003106405, WO 2005087751, WO 2005087215, WO 2005016876, US 2006281729, WO 2008119772, WO 2006074950, WO 2007077004, WO 2005049585, US 6251928 und US 6150416.

Die WO 2012107153 offenbart peptidische Verbindungen sowie auch Pepstatin als Cathepsin D Antagonisten, die zur Behandlung von Arthrose verwendet werden können und die dazu intraartikulär appliziert werden können.

Die bekannten Cathepsin D Inhibitoren und die beiden Modellverbindungen Pepstatin und Ritonavir hemmen zwar wirksam die Cathepsin D Aktivität, jedoch weisen sie eine recht geringe Selektivität gegenüber anderen Aspartylproteasen auf. Die Rolle des Renin-Angiotensin Systems (RAS) bei der Regulation des Blutdrucks und des Flüssigkeits- und Elektrolythaushalts (Oparil, S. et al., N. Engl. J. Med. 1974; 291: 381-401/446-57) und die Wirksamkeit von Renin- und Pepsininhibitoren bei Erkrankungen des Herzkreislauf- und Gefäßsystems ist hinreichend bekannt und so ist insbesondere bei oraler bzw. systemischer Applikation dieser wenig selektiven Cathepsin D Inhibitoren mit zahlreichen Nebenwirkungen zu rechnen und auch bei lokaler Applikation ist durch die zu erwartende Diffusion der Verbindungen ins Blut mit systemischen Komplikationen zu rechnen.

Daneben weisen gerade peptidische Verbindungen im Plasma, Synovialflüssigkeit und Flüssigkeiten anderer Kompartimente in der Regel eine geringe Stabilität auf und sie werden sehr schnell metabolisch abgebaut, so dass eine geringe Verweildauer im Blut, in der Gelenkkapsel und anderen Kompartimenten zu erwarten ist.

So untersuchten Powell, M. F. et al. (J. Pharm. Sciences, Vol. 81, No. 8, 731-735, 1992) die Stabilität von peptidischen Verbindungen in vereinigtem (gepooltem) humanem Serum und in vereinigter (gepoolter) Synovialflüssigkeit von Patienten mit rheumatischer Arthritis (s. S. 731, rechte Spalte, vorletzter Absatz). In den Tabellen 1 und 2 offenbarten Powell et al., dass die meisten der getesteten modifizierten und nicht modifizierten Peptide mit einer Länge von 10 bis 25 Aminosäuren eine Halbwertszeit von weniger als einer Stunde in den getesteten Medien, humanes Plasma (HS), Synovialflüssigkeit (SF), fötales Kälberserum (FCS) oder Maus-Leberhomogenat (MLH) aufweisen (siehe Seite 735, rechte Spalte, letzter Absatz). Die Stabilität der peptidischen Verbindungen in vereinigtem HumanSerum und vereinigter Synovialflüssigkeit von Patienten mit Arthritis ist dabei grundsätzlich ähnlich gering (siehe Seite 733).

Vor dem Hintergrund der geringen Stabilität und der geringen Verweildauer peptidischer Verbindungen im Plasma und wegen der oben beschriebenen zu erwartenden Nebenwirkungen kommt eine orale oder systemische Verabreichung von peptidischen Cathepsin D Inhibitoren zur Behandlung von Arthrose nicht in Betracht.

Auch eine intraartikuläre Applikation peptidischer Verbindungen kommt aufgrund der zu erwartenden geringen Halbwertszeit in Synovialflüssigkeit, aber insbesondere aufgrund der zu erwartenden geringen Verweildauer in der Gelenkkapsel (Diffusion über die Synovialmembran und Degradation) und aufgrund der durch die Diffusion ins Plasma zu erwartenden systemischen Nebenwirkungen für den Fachmann in der Regel nicht in Frage.

Vor allem wären aufgrund der nach Powell et al. (1992) geringen Halbwertszeit peptidischer Verbindungen von wenigen Stunden häufige intraartikuläre Injektionen notwendig. Injektionen in den Gelenkspalt sind jedoch für den Patienten mit Schmerzen und einem beträchlichen Infektionsrisiko verbunden und deshalb sollten solche Injektionen nicht häufiger als im Abstand von zwei bis vier Wochen erfolgen.

Aufgabe der vorliegenden Erfindung war es deshalb, neue Arzneimittel und pharmazeutische Zubereitungen zu finden, die zur Vorbeugung und Behandlung von Arthrose eingesetzt werden können und bei lokaler bzw. intraartikulärer Applikation in Synovialflüssigkeit ausreichend stabil sind und nur in geringem Maße durch die Synovialmembran ins Plasma diffundieren und damit eine hohe Verweildauer in der Gelenkkapsel aufweisen, so dass nach Injektion die Wirkstoffkonzentration über einen möglichst langen Zeitraum im therapeutisch wirksamen Bereich bleibt.

### Zusammenfassung der Erfindung

Überraschenderweise wurde gefunden, dass Pepstatin, trotz hoher Clearance bei intravenöser oder oraler Applikation, bei intraartikulärer Injektion einer Suspension über einen längeren Zeitraum in einer Konzentration deutlich über dem IC₅₀ und damit in pharmakologisch wirksamer Konzentration in der Gelenkkapsel bzw. in der Synovialflüssigkeit verbleibt. Außerdem zeigt Pepstatin, trotz seiner peptidischen Struktur, eine überraschend hohe Stabilität in Synovialflüssigkeit (siehe Beispiel) und damit überschreitet Pepstatin selbst die längsten von Powell et al. gemessenen Stabilitäten peptidischer Verbindungen in Synovialflüssigkeit deutlich (siehe Powell et al. Tabelle II auf Seite 733: Nr. 8 Pertussis-Toxin mit einer Halbwertzeit von knapp zwei Tagen in Synovialflüssigkeit, Nr. 15 Myobacterium leprae mit einer Halbwertzeit von etwa siebeneinhalb Stunden, alle weiteren peptidischen Verbindungen mit Halbwertzeiten von wenigen Minuten) und erst diese überraschend hohe Stabilität und die überraschend hohe Verweildauer in der Gelenkkapsel ermöglichen eine medizinisch relevante Verwendung von Pepstatin bei der Behandlung von Arthrose, da Pepstatin eben nicht, wie nach Powell et al. zu erwarten war, nur wenige Stunden mit klinisch relevanten Spiegeln in der Gelenkkapsel erhalten bleibt, sondern mehr als zwei Wochen in einer Konzentration deutlich über dem IC₅₀ vorliegt.

Damit eignet sich Pepstatin überraschenderweise für Arzneimittel und pharmazeutische Zubereitungen, die zur Vorbeugung und Behandlung von Arthrose lokal bzw. intraartikulär appliziert werden und über einen langen Zeitraum mit hohen Spiegeln erhalten bleiben, so dass die erfindungsgemäßen Arzneimittel und pharmazeutische Zubereitungen nur höchstens wöchentlich intraartikulär appliziert werden müssen, bevorzugt in Intervallen von einem bis mehreren Monaten.

Pepstatin hemmt hochwirksam Cathepsin D und bei der intraartikulären Applikation zur Behandlung von Arthrose ist mit geringen Nebenwirkungen zu rechnen, da Pepstatin bei intraartikulärer Applikation aufgrund seiner hohen Verweildauer in der Gelenkkapsel und langsamen Freisetzung aus dem Synovium nur geringe systemische Spiegel (Plasmaspiegel) erreicht.

Insbesondere die hohe Verweildauer von Pepstatin in der Gelenkkapsel ist überraschend und therapeutisch wertvoll, da ausgehend von den Untersuchungen von beispielweise Powell et al. (1992) eine geringe Stabilität peptidischer Verbindungen in Synovialflüssigkeit zu erwarten gewesen wäre und man darüber hinaus mit einer hohen Freisetzung von peptidischen Verbindungen geringer Größe aus dem Synovium ins Plasma gerechnet hätte, beides Vorgänge, die zu einer geringen Verweildauer in der Gelenkkapsel führen würden. Überraschenderweise verfügt jedoch Pepstatin wider Erwarten über eine lange Verweildauer in der Gelenkkapsel, da sowohl seine Stabilität in Synovialflüssigkeit hoch, als auch die Freisetzung aus dem Synovium offensichtlich sehr gering ist.

Pepstatin enthält mehrere Chiralitätszentren, sodass auch die Verwendung der optisch aktiven Formen (Stereoisomeren), der Enantiomeren, Racemate, Diastereomeren sowie Hydrate und Solvate von Pepstatin Gegenstand der Erfindung ist.

Unter pharmazeutisch oder physiologisch unbedenklichen Derivaten versteht man z.B. Salze von Pepstatin, als auch sogenannte Prodrug-Verbindungen. Unter Prodrug-Verbindungen versteht man mit z.B. Alkyl- oder Acylgruppen (siehe auch nachstehende Amino- und Hydroxyschutzgruppen), Zuckern oder Oligopeptiden abgewandelte Pepstatin-Derivate, die im Organismus rasch zu den wirksamen Pepstatin-Molekülen gespalten oder freigesetzt werden. Hierzu gehören auch bioabbaubare Polymerderivate von Pepstatin, wie dies z.B. in Int. J. Pharm. 115 (1995), 61-67 beschrieben ist.

Pepstatin lässt sich in seiner endgültigen Nichtsalzform verwenden. Andererseits umfaßt die vorliegende Erfindung auch die Verwendung von Pepstatin in Form seiner pharmazeutisch unbedenklichen Salze, die von verschiedenen organischen und anorganischen Basen nach fachbekannten Vorgehensweisen abgeleitet werden können. Pharmazeutisch unbedenkliche Salzformen von Pepstatin werden größtenteils konventionell hergestellt. Da Pepstatin eine Carbonsäuregruppe enthält, läßt sich eines seiner geeigneten Salze dadurch bilden, daß man Pepstatin mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze von Pepstatin zählen ebenfalls dazu.

Weiterhin zählen zu den Basensalzen von Pepstatin Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll.

Bevorzugt unter den oben genannten Salzen sind Ammonium; die Alkalimetallsalze Natrium und Kalium, sowie die Erdalkalimetalsalze Calcium und Magnesium. Zu Salzen von Pepstatin, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminöethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Wie erwähnt werden die pharmazeutisch unbedenklichen Basenadditionssalze von Pepstatin mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von Pepstatin werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Im Hinblick auf das oben Gesagte sieht man, daß unter dem Ausdruck "pharmazeutisch unbedenkliches Salz" im vorliegenden Zusammenhang ein Wirkstoff zu verstehen ist, der Pepstatin in der Form eines seiner Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Unter Solvaten von Pepstatin werden Anlagerungen von inerten Lösungsmittelmolekülen Pepstatin verstanden, die sich aufgrund ihrer gegenseitigen Anziehungskraft ausbilden. Solvate sind beispielsweise Hydrate, wie Monohydrate oder Dihydrate oder Alkoholate, d.h. Additionsverbindungen mit Alkoholen wie beispielsweise mit Methanol oder Ethanol.

Es wurde gefunden, dass Pepstatin gut verträglich ist und wertvolle pharmakologische Eigenschaften besitzt, da es Aspartylproteasen und insbesondere Cathepsin D selektiv inhibiert.

Unter physiologischen und/oder pathophysiologischen Zuständen werden physiologische und/oder pathophysiologische Zustände verstanden, die medizinisch relevant sind, wie beispielsweise Krankheiten bzw. Erkrankungen und medizinische Störungen, Beschwerden, Symptome oder Komplikationen und dergleichen, insbesondere Krankheiten.

Schmerz ist eine komplexe Sinneswahrnehmung, die als akutes Geschehen den Charakter eines Warn- und Leitsignals aufweist, als chronischer Schmerz diesen aber verloren hat und in diesem Fall (als Chronisches Schmerzsyndrom) heute als eigenständiges Krankheitsbild gesehen und behandelt werden soll. Als Hyperalgesie wird in der Medizin eine übermäßige Schmerzempfindlichkeit und Reaktion auf einen üblicherweise schmerzhaften Reiz hin bezeichnet. Reize, die Schmerzen auslösen können, sind beispielsweise Druck, Wärme, Kälte oder Entzündungen. Die Hyperalgesie ist eine Form der Hyperästhesie, dem Oberbegriff für eine übermäßige Empfindlichkeit auf einen Reiz hin. Als Allodynie wird in der Medizin eine Schmerzempfindung bezeichnet, die durch Reize ausgelöst wird, welche üblicherweise keinen Schmerz verursachen.

Pepstatin zeigt eine vorteilhafte biologische Aktivität, die in Enzym-Assays und Tierversuchen, wie in den Beispielen beschrieben, leicht nachweisbar ist. In derartigen auf Enzymen basierenden Assays zeiget und bewirkt Pepstatin einen inhibierenden Effekt, der gewöhnlich durch IC₅₀-Werte in einem geeigneten Bereich, bevorzugt im mikromolaren Bereich und besonders bevorzugt im nanomolaren Bereich dokumentiert wird.

Pepstatin kann an Menschen oder Tiere, insbesondere Säugetiere, wie Affen, Pferde, Hunde, Katzen, Ratten oder Mäuse verabreicht werden und bei der therapeutischen Behandlung des menschlichen oder des tierischen Körpers sowie bei der Bekämpfung der oben aufgeführten Krankheiten verwendet werden. Es kann weiterhin als Diagnostikum oder als Reagenz Verwendung finden.

Pepstatin kann zur Herstellung pharmazeutischer Zubereitungen zur intraartikulären Applikation verwendet werden, insbesondere auf nichtchemischem Wege. Hierbei wird es zusammen mit mindestens einem festen, flüssigen und/oder halbflüssigen Träger- und/oder Hilfsstoff und gegebenenfalls in Kombination mit einem oder mehreren weiteren Wirkstoff(en) in eine geeignete Dosierungsform gebracht.

Die erfindungsgemäßen pharmazeutischen Zubereitungen können als Arzneimittel in der Human- oder Veterinärmedizin verwendet werden. Der Patient oder Wirt kann jeglicher Säugerspezies angehören, z.B. einer Primatenspezies, besonders Menschen; Nagetieren, einschließlich Mäusen, Ratten und Hamstern; Kaninchen; Pferden, Rindern, Hunden, Katzen usw.. Tiermodelle sind für experimentelle Untersuchungen von Interesse, wobei sie ein Modell zur Behandlung einer Krankheit des Menschen zur Verfügung stellen.

Als Trägersubstanzen kommen organische oder anorganische Stoffe in Frage, die sich für die intraartikuläre Applikation eignen und mit den erfindungsgemäßen Verbindungen nicht reagieren. Welche Hilfsstoffe für die gewünschte Arzneimittelformulierung geeignet sind, ist dem Fachmann auf Grund seines Fachwissens geläufig. Neben Lösungsmitteln z.B. Wasser, physiologische Kochsalzlösung oder Alkohole wie z.B. Ethanol, Propanol oder Glycerin, Zuckerlösungen wie Glucose oder Mannitlösungen oder einer Mischung der genannten Lösungsmittel und anderen Wirkstoffträgern können Stabilisatoren und/oder Netzmittel, Emulgatoren, Salze zur Beeinflussung des osmotischen Druckes, Antioxidantien, Dispergiermittel, Entschäumer, Puffersubstanzen, Konservierungsmittel oder Lösungsvermittler verwendet werden. Falls erwünscht, können erfindungsgemäße Zubereitungen oder Medikamente einen oder mehrere weitere Wirkstoffe enthalten, beispielsweise ein oder mehrere Vitamine oder Wirkstoffe, die bei der Pophylaxe und/oder Behandlung der obengeannten medizinischen Indikationen wirksam sind.

Falls erwünscht, können erfindungsgemäße Zubereitungen oder Medikamente einen oder mehrere weitere Wirkstoffe und/oder einen oder mehrere Wirkverstärker (Adjuvantien) enthalten.

Die Begriffe "pharmazeutische Formulierung" und "pharmazeutische Zubereitung" werden im Rahmen der vorliegenden Erfindung als Synonyme verwendet.

Wie hier verwendet bezieht sich "pharmazeutisch verträglich" auf Arzneimittel, Präzipitationsreagezien, Trägerstoffe, Hilfsstoffe, Stabilisatoren, Lösungsmittel und sonstige Agenzien, die die Verabreichung der daraus erhaltenen pharmazeutischen Zubereitungen ohne unerwünschte physiologische Nebenwirkungen an ein Säugetier ermöglichen.

Bei pharmazeutischen Zubereitungen zur parenteralen Verabreichung besteht die Forderung nach Isotonie, Euhydrie sowie nach Verträglichkeit und Sicherheit der Formulierung (geringe Toxizität), der eingesetzten Hilfsstoffe und des Primärpackmittels. Überraschenderweise hat Pepstatin vorzugsweise den Vorteil, dass eine direkte Verwendung möglich ist und vor Verwendung in pharmazeutischen Formulierungen keine weiteren Reinigungsschritte zum Entfernen toxikologisch bedenklicher Agenzien, wie beispielsweise hohe Konzentrationen organischer Lösungsmittel oder anderer toxikologisch bedenklicher Hilfsstoffe, erforderlich sind.

Erfindungsgemäße pharmazeutische Zubereitungen zur intraartikulären Applikation können Pepstatin in präzipitierter nicht-kristalliner, präzipitierter kristalliner oder in gelöster oder suspendierter Form, sowie gegebenenfalls Träger- und/oder Hilfsstoffe und/oder weitere pharmazeutische Wirkstoffe enthalten.

Vorzugsweise ermöglicht es Pepstatin, hochkonzentrierte Formulierungen herzustellen, ohne dass es zu ungünstigen unerwünschten Aggregationen von Pepstatin kommt. So lassen sich mithilfe Pepstatin mit wässrigen Lösungsmitteln oder in wässrigen Medien applikationsfertige Lösungen mit hohem Wirkstoffanteil herstellen.

Pepstatin und/oder dessen physiologisch unbedenkliche Salze und Solvate können auch lyophilisiert und die erhaltenden Lyophilisate beispielsweise zur Herstellung von Injektionspräparaten zur intraartikulären Applikation verwendet werden.

Wässrige Zubereitungen zur intraartikulären Applikation können hergestellt werden, indem man Pepstatin in einer wässrigen Lösung löst oder suspendiert und gegebenenfalls Hilfsstoffe zufügt. Zweckmäßigerweise wird hierzu einer Lösung oder Suspension mit einer definierten Konzentration an Pepstatin mit definierten Volumina an Stammlösungen, die die genannten weiteren Hilfsstoffe in definierter Konzentration enthalten, versetzt und ggf. mit Wasser auf die vorberechnete Konzentration verdünnt. Alternativ können die Hilfsstoffe in fester Form zugesetzt werden. Die erhaltene wässrige Lösung oder Suspension kann anschließend mit den jeweils erforderlichen Mengen an Stammlösungen und/oder Wasser versetzt werden. Zweckmäßigerweise kann Pepstatin auch direkt in einer alle weiteren Hilfsstoffe enthaltenden Lösung gelöst oder suspendiert werden.

Vorteilhaft können Pepstatin enthaltenden Lösungen oder Suspensionen mit einem pH-Wert von 4 bis 10, bevorzugt mit einem pH-Wert von 5 bis 9, und einer Osmolalität von 250 bis 350 mOsmol/kg hergestellt werden. Die pharmazeutische Zubereitung kann somit weitgehend schmerzfrei intraartikulär direkt verabreicht werden. Daneben können der Zubereitung zur intraartikulären Applikation auch Infusionslösungen, wie z.B. Glukoselösung, isotonische Kochsalzlösung oder Ringerlösung, die auch weitere Wirkstoffe enthalten können, zugesetzt werden, sodass auch größere Wirkstoffmengen appliziert werden können.

Pepstatin ist physiologisch gut verträglich, leicht herstellbar, exakt dosierbar und vorzugsweise über die Dauer der Lagerung sowie des Transports und bei mehrfachen Einfrier- und Auftauvorgängen hinsichtlich Gehalt, Zersetzungsprodukten und Aggregaten stabil. Es kann bei Kühlschranktemperatur (2-8°C) und bei Raumtemperatur (23-27°C) und 60 % relativer Luftfeuchtigkeit (r.F.) vorzugsweise über einen Zeitraum von mindestens drei Monaten bis zu zwei Jahren stabil gelagert werden.

Beispielsweise kann Pepstatin durch Trocknung stabil gelagert und bei Bedarf durch Lösung oder Suspension in eine gebrauchsfertige pharmazeutische Zubereitung überführt werden. Mögliche Methoden zur Trocknung sind zum Beispiel, ohne auf diese Beispiele beschränkt zu sein, Stickstoffgastrocknung, Vakuumofen-Trocknung, Lyophilization, Waschen mit organischem Lösungsmittel und anschließende Lufttrocknung, Flüssigbett-Trocknung, Wirbelschichttrocknung, Sprühtrocknung, Walzentrocknung, Lagentrocknung; Lufttrocknung bei Raumtemperatur und weitere Methoden.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder angestrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat: verbesserte Heilbehandlung, Heilung, Prävention oder Beseitigung einer Krankheit, eines Krankheitsbildes, eines Krankheitszustandes, eines Leidens, einer Störung oder Verhinderung von Nebenwirkungen oder auch die Verminderung des Fortschreitens einer Krankheit, eines Leidens oder einer Störung. Die Bezeichnung "therapeutisch wirksame Menge" umfasst auch die Mengen, die wirkungsvoll sind, die normale physiologische Funktion zu erhöhen.

Bei Verwendung von erfindungsgemäßen Zubereitungen oder Medikamenten zur intraartikulären Applikation wird Pepstatin und/oder dessen physiologisch unbedenklichen Salze und Solvate in der Regel analog zu bekannten, käuflich erhältlichen Zubereitungen oder Präparaten verwendet. Die Dosierung hängt dabei vom Alter, Geschlecht, Gewicht und Gesundheitszustand und Konstitution des Patienten ab, sowie der Schwere seiner Erkrankung und weiteren individuellen Faktoren.

Die erfindungsgemäßen pharmazeutischen Zubereitungen zur intraartikulären Applikation werden vorzugsweise wöchentlich bis jährlich, besonders bevorzugt 14-tägig bis halbjährlich, ganz besonders bevorzugt monatlich bis vierteljährlich intraartikulär verabreicht.

Deshalb ist ein Gegenstand der vorliegenden Erfindung eine pharmazeutische Zubereitung enthaltend Pepstatin und/oder eines seiner physiologisch unbedenklichen Salze, Solvate und Stereoisomere, einschließlich deren Mischungen in allen Verhältnissen und weitere Träger- und/oder Hilfsstoffe, zur Verwendung als Medikament bei der Behandlung und/oder Prophylaxe von physiologischen und/oder pathophysiologischen Zuständen, ausgewählt aus der Gruppe bestehend aus Arthrose, traumatischen Knorpelverletzungen, Arthritis, Schmerz, Allodynie oder Hyperalgesie, wobei die pharmazeutische Zubereitung wie nachfolgend intraartikulär appliziert wird:
a) wöchentlich bis jährlich,
b) 14-tägig bis halbjährlich oder
c) monatlich bis vierteljährlich.

Ein bevorzugter Gegenstand der vorliegenden Erfindung ist auch eine vorstehende pharmazeutische Zubereitung, wobei die pharmazeutische Zubereitung wenigstens einen weiteren Arzneimittelwirkstoff enthält.

Ein besonders bevorzugter Gegenstand der vorliegenden Erfindung ist eine vorstehende pharmazeutische Zubereitung, zur Verwendung als Medikament bei der Behandlung und/oder Prophylaxe von Arthrose.

Ein weiterer Gegenstand der vorliegenden Erfindung ist Pepstatin und/oder eines seiner physiologisch unbedenklichen Salze, Solvate und Stereoisomere, einschließlich deren Mischungen in allen Verhältnissen zur Verwendung als Medikament bei der Behandlung und/oder Prophylaxe von physiologischen und/oder pathophysiologischen Zuständen, ausgewählt aus der Gruppe bestehend aus Arthrose, traumatische Knorpelverletzungen, Arthritis, Schmerz, Allodynie und Hyperalgesie, wobei Pepstatin und/oder eines seiner physiologisch unbedenklichen Salze, Solvate und Stereoisomere, einschließlich deren Mischungen in allen Verhältnissen wie nachfolgend intraartikulär appliziert wird:
a) wöchentlich bis jährlich,
b) 14-tägig bis halbjährlich oder
c) monatlich bis vierteljährlich.

Ein bevorzugter Gegenstand ist vorstehendes Pepstatin und/oder eines seiner physiologisch unbedenklichen Salze, Solvate und Stereoisomere, einschließlich deren Mischungen in allen Verhältnissen zur Verwendung als Medikament bei der Behandlung und/oder Prophylaxe von Arthrose.

Jedoch hängen die individuelle Dosierung und Verabreichungsintervalle für einen Patienten auch von einer großen Zahl individueller Faktoren ab, wie beispielsweise von der Wirksamkeit der jeweils verwendeten Verbindung, vom Alter, Körpergewicht, allgemeinen Gesundheitszustand, Geschlecht, Ernährung, vom Verabreichungszeitpunkt und -weg, von der Ausscheidungsrate, von der Kombination mit anderen Arzneimitteln und von der Schwere und Dauer der jeweiligen Erkrankung.

Ein Maß für die Aufnahme eines Arzneimittelwirkstoffs in einen Organismus ist seine Bioverfügbarkeit. Wird der Arzneimittelwirkstoff in Form einer Injektionslösung dem Organismus intraartikulär zugeführt, so liegt seine absolute Bioverfügbarkeit, d.h. der Anteil des Pharmakons, der unverändert in den Gelenkspalt gelangt, bei 100%. Daten zur Pharmakokinetik, d.h. zur Bioverfügbarkeit können analog zu der Methode von J. Shaffer et al. (J. Pharm. Sciences, 88 (1999), 313-318) erhalten werden.

Weiterhin lassen sich solche Arzneimittel mit einem der im pharmazeutischen Fachgebiet allgemein bekannten Verfahren herstellen.

Arzneimittel lassen sich zur Verabreichung auf intraartikulärem Wege anpassen. Solche Arzneimittel können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

Die intraartikuläre Applikation hat den Vorteil, dass die erfindungsgemäße Verbindung direkt in die Nähe des Gelenkknorpels in die Synovialflüssigkeit appliziert wird und von dort auch in das Knorpelgewebe hineindiffundieren kann. Erfindungsgemäße pharmazeutische Zubereitungen können somit auch direkt in den Gelenkspalt injiziert werden und so direkt am bestimmungsgemäßen Wirkort ihre Wirkung entfalten. Die erfindungsgemäße Verbindung eignet sich auch zur Herstellung von intraartikulär zu applizierenden Arzneimitteln mit kontrollierter, gleichmäßiger und/oder verzögerter Wirkstofffreisetzung (slow-release, sustained-release, controlled release). Somit eignet sich Pepstatin auch zur Herstellung von Depot-Formulierungen, die für den Patienten vorteilhaft sind, da eine Applikation nur in größeren Zeitintervallen notwendig ist.

Zu den an die intraartikuläre Verabreichung angepassten Arzneimitteln gehören wässrige und nichtwässrige sterile Injektionslösungen, die Antioxidantien, Puffer, Bakteriostatika und Solute, durch die die Formulierung isotonisch mit der Synovialflüssigkeit des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Pepstatin lässt sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Pepstatin kann auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin kann Pepstatin an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate, Polymilchcoglykolsäure, Polymere wie Konjugate zwischen Dextran und Methacrylate, Polyphosphoester, verschiedene Polysaccharide und Polyamine sowie Poly-ε-caprolacton, Albumin, Chitosan, Collagen oder modifizierte Gelatine und quervernetzte oder amphipatische Blockcopolymere von Hydrogelen, gekoppelt sein.

Es versteht sich, dass die erfindungsgemäßen Arzneimittel neben den obigen besonders erwähnten Bestandteilen andere im Fachgebiet übliche Mittel mit Bezug auf die jeweilige Art der pharmazeutischen Formulierung enthalten können.

Weiterhin können die erfindungsgemäßen Arzneimittel verwendet werden, um bei gewissen bekannten Therapien additive oder synergistische Effekte bereitzustellen, und/oder können dazu verwendet werden, um die Wirksamkeit gewisser existierender Therapien wiederherzustellen.

Die erfindungsgemäßen pharmazeutischen Zubereitungen können neben Pepstatin noch weitere Arzneimittelwirkstoffe enthalten, z.B. zur Verwendung bei der Behandlung von Arthrose andere Cathepsin D-Inhibtioren, NSAIDS, Cox-2-Inhibitoren, Glucocorticoide, Hyaluronsäure, Azathioprin, Methotrexat, anti-CAM Antikörper, wie beispielweise anti-ICAM-1 Antikörper und/oder FGF-18. Zur Behandlung der anderen genannten Erkrankungen können die erfindungsgemäßen pharmazeutischen Zubereitungen neben Pepstatin noch weitere Arzneimittelwirkstoffe, die dem Fachmann bei deren Behandlung bekannt sind, enthalten.

Auch ohne weitere Ausführungsformen wird davon ausgegangen, dass ein Fachmann die obige Beschreibung im weitesten Umfang nutzen kann.

Sofern nichts anderes angegeben ist, bedeuten Prozentangaben Gewichtsprozent. Alle Temperaturen sind in Grad Celsius angegeben. "Übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, filtriert, dampft ein und reinigt durch Chromatographie an Kieselgel und /oder durch Kristallisation. Rf-Werte an Kieselgel; Massenspektrometrie: El (Elektronenstoßionisation): M⁺, FAB (Fast Atom Bombardment): (M+H)⁺, THF (Tetrahydrofuran), NMP (N-Methlpyrrolidon), DMSO (Dimethlysulfoxid), EE (Ethylacetat), MeOH (Methanol), DC (Dünnschichtchromatographie)

Pepstatin wurde synthetisiert und charakterisiert. Die Herstellung und Charakterisierung von Pepstatin ist jedoch auch auf anderen Wegen für den Fachmann durchführbar.

### Beispiel 1: Pepstatin - ein peptidischer Cathepsin D Inhibitor

**Tabelle 1**

| Struktur | Cath D IC₅₀ [M] gemäß Beispiel 2 | Cath D IC₅₀ [M] gemäß Beispiel 3 |
|---|---|---|
| 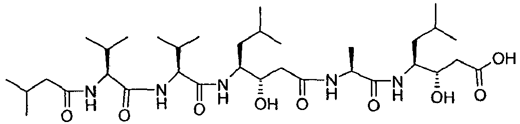 | 1.6-1,90E-09 | 0.69-2,40E-09 |
| Pepstatin: (3S,4S)-3-Hydroxy-4-[(S)-2-((3S,4S)-3-hydroxy-6-methyl-4-{(S)-3-methyl-2-[(S)-3-methyl-2-(3-methyl-butyrylamino)-butyrylamino]-butyrylamino}-heptanoylamino)-propionylamino]-6-methyl-heptansäure | | |

sowie dessen physiologisch unbedenkliche Salze, Derivate, Solvate, Prodrugs und Stereoisomere, einschließlich deren Mischungen in allen Verhältnissen.

Pepstatin zeichnet sich darüber hinaus durch eine hohe Selektivität für Cathepsin D gegenüber Renin (IC₅₀ >10000nM), gute Knorpelpenetration und keine meßbare Toxizität bzw. Genotoxizität aus.

### Beispiel 2: In-vitro Fluoreszenz-Assay zur Identifizierung von Cathepsin D Inhibitoren

Zur Identifizierung von Modulatoren der Cathepsin D Aktivität wurde ein kontinuierlicher enzymatischer Test mit einem synthetischen Peptid, das eine fluoreszierende Gruppe (MCA=(7-methoxycoumarin-4-yl)acetyl) trägt, die durch Energietransfer von einer Dpn (2,4 dinitrophenyl)-Gruppe am selben Molekül gequencht ist, in 384-well-nb-Mikrotiterplatten von Greiner durchgeführt. Die Spaltung des peptidischen Substrats durch Cathepsin D bewirkt einen Anstieg in der Fluoreszenzintensität. Zur Bestimmung der Wirksamkeit von Substanzen wurde der zeitabhängige Anstieg in der Fluoreszenzintensität in Gegenwart der Substanz mit der zeitabhängigen Fluoreszenzzunahme in Abwesenheit von Substanzen verglichen. Als Referenzsubstanz diente Pepstatin A (Sigma-Aldrich). Als Substrat wurde MCA-GKPILFFRLK(Dnp)d-R-NH₂ (Enzo Life Sciences, Lörrach) verwendet. Als Enzym wurde aus der humanen Leber isoliertes Cathepsin D (Sigma-Aldrich) in einer finalen Konzentration von 1.4 nM eingesetzt. Der Test wurde in 100 mM Natrium-Acetat-Puffer, 1.25 % (v/v) DMSO, 0.25 % (w/v) Chaps, pH 5.5 durchgeführt. Zu je 4 µl Cathepsin D-Lösung wurden je 2 µl Substanzlösung mit seriell verdünnter Substanzkonzentration zugegeben und 10 min bei Raumtemperatur inkubiert. Die Reaktion wurde gestartet durch Zugabe von 2 µl Substrat-Lösung (finale Konzentration 5 µM). Nach Durchführung einer Startpunkt-Fluoreszenzmessung (Anregungswellenlänge 340 nm/Emissionswellenlänge 450 nm) mit einem Envision Multilabel reader (Perkin Elmer) wurde die Reaktion 60 min bei Raumtemperatur inkubiert. Anschließend wurde die Menge des während der Reaktionszeit abgespaltenen Peptidfragments durch Bestimmung der Zunahme der Fluoreszenzintensität bei 450 nm (Anregungswellenlänge 340 nm) gemessen.

### Ergebnis: Pepstatin hemmt Cathpsin D im nanomolaren Bereich (siehe Tabelle 1)

### Beispiel 3: Knorpel-Explant-Assay

Um den Effekt von potentiellen Cathepsin D Inhibitoren auf Knorpelabbau zu untersuchen wird ein pH-induziertes Modell basierend auf bovinen Explantaten verwendet. Dabei wird der pH-Wert des Mediums, in dem die Explantate kultiviert werden, dem pathophysiologischen pH-Wert eines arthrotischen Knies angepasst. Dieser pH-Wert liegt bei pH 5,5. In diesem *ex vivo* Modell werden anschließend potentielle Cathepsin D Inhibitoren auf ihre Wirkung hinsichtlich einer Arretierung des Knorpelabbauprozesses untersucht. Wird der Knorpel zerstört, werden Glycosaminoglycane (GAGs) in den Zellkulturüberstand abgegeben. Die Menge der freigesetzten GAGs lässt sich quantitativ mit Hilfe des DMMB (Dimethylmethylenblau-Hydrochlorid) bestimmen. Beim Nachweis sulfatierter GAGs mit Dimethylmethylenblau-Hydrochlorid macht man sich die Abnahme der Absorption bei 633 nm zu Nutze. Da auch bei sehr niedrigen GAG-Konzentrationen gearbeitet werden kann, fällt auch nach langer Inkubation von DMMB mit GAG kein Farbstoff/GAG-Komplex aus, wie das bei anderen Messmethoden mitunter schon nach kurzer Zeit passiert. Zur Bestimmung der Konzentration wird eine Eichgerade mit Chondroitinsulfat mitgeführt. Anhand der GAG-Werte lässt sich ein 1C₅₀-Wert errechnen, d.h. eine Konzentration bei der eine Substanz 50% ihrer Wirkung zeigt.

### Lösungen:

### Inkubationsmedium, pH 7,4:

DMEM ohne FBS, Zugabe von 1 % Pen/Strep und 30 µg/ml Ascorbinsäure, das Medium wird nicht gelagert.

### Inkubationsmedium, pH 5,5:

DMEM ohne FBS, der pH-Wert wird durch Zugabe von MES eingestellt und am pH-Meter kontrolliert, Zugabe von 1% Pen/Strep und 30 µg/ml Ascorbinsäure.

### Lösungen für die GAG-Messung:

### DMMB-Färbelösung (V = 500 ml):

8 mg DMMB (Dimethylmethylenblau) in 2,5 ml Ethanol lösen + 1 g Natriumformiat + 1 ml Ameisensäure, mit bidest. Wasser ad 500 ml auffüllen

### Inkubationsmedium: FBS (Medium ohne FBS)

### Chondroitinsulfat-Lösungen (Standardkurve)

Ansatz von Standardlösungen mit folgenden Konzentrationen: 50 µg/ml; 25 µg/ml; 12,5 µg/ml; 6,25µg/ml; 3,125 µg/ml; 1,5 6µg/ml; 0,78 µg/ml sowie eine Leerkontrolle des Mediums. Der Ansatz der Standardlösung erfolgt in dem Medium, mit welchem auch der Versuch durchgeführt wurde.

### 1.) Durchführung: pH induzierter Knorpelabbau von bovinen Explantaten

Die bovinen Explantate werden zunächst präpariert. Die Induktion des Knorpelabbaus wird in 96-Multiwellplatten durchgeführt. Dabei wird ein Explantat pro well kultiviert. Es erfolgt die Zugabe von je 200 µl DMEM (Inkubationsmedium pH 5,5) ohne FBS + 30 µg/ml Ascorbinsäure. Also Negativkontrolle werden Explantate (n= 4) bei pH 7,4 (ohne FBS) inkubiert. Diese Kontrolle geht nicht in die Berechnung der Daten mit ein, sondern stellt sicher dass die pH-Wert Änderung den gewünschten Effekt auf die Freisetzung von GAG hat. An dieser Stelle erfolgt die Zugabe der zu testenden Substanzen. Es findet keine Vorinkubation der Explantate statt.Die Explantate werden mit den entsprechenden Substanzen 3 Tage im Brutschrank bei 37°C und 7,5% CO₂ kultiviert.

### 2.) Inkubationsablauf

Um den Effekt von Cathepsin D Inhibitoren auf die Freisetzung von GAG (Glycosaminoglycan) zu untersuchen, werden die Substanzen in der gewünschten Konzentration eingesetzt und für 3 Tage kultiviert. Dabei werden die zu testenden Verbindungen in einem ersten Experiment in einer Konzentration von 1 µM und 1% DMSO getestet. Substanzen die einen Effekt von >50% auf die Freisetzung von GAG haben (das entspricht <50% der Kontrolle im Assay Explorer), werden in einem nächsten Experiment bei 100 nM und 1% DMSO gestestet. Substanzen, die unter diesen Bedingungen einen Effekt von >50% auf die Freisetzung von GAG haben (das entspricht <50% der Kontrolle im Assay Explorer), werden in einer Konzentrations-Wirkungs-Beziehung getestet. Dabei werden die Verbindungen in den folgenden Konzentrationen untersucht: 30 µM, 10 µM, 3 µM, 1 µM, 0.3 µM, 0.1 µM, 0.03 µM, 0.01 µM.

Als Positivkontrolle wird Pepstatin A mit einer Konzentration von 0,01 µM verwendet. Das Wirkungsfenster (assay window) ist definiert durch die Kontrolle (pH 5,5), definiert als 0% Effekt und die Kontrolle pH 5,5 + 0,01 µM Pepstatin A, definiert als 100% Effekt. Nach 3 Tagen Inkubation werden die Zellkulturüberstände gesammelt und bei -20°C gelagert oder direkt vermessen. Dabei wird photometrisch die Menge an freigesetztem GAG gemessen.

Berichtet werden für Konzentrationen von 1 µM und 100 nM der Effekt (1 Wert) der jeweiligen Substanz in % bezogen auf die Positivkontrolle (pH 5,5 + 0,01 µM Pepstatin A) und die Negativkontrolle (pH 5,5). Der Wert stellt den Mittelwert aus 4 Replikaten da. Bei der Ermittlung einer Konzentrations-Wirkungs-Beziehung wird ein IC₅₀-Wert an die Datenbank berichtet (Assay Explorer).

### 4.) Messung

Die Zellkulturüberstände (200 µl) werden entweder direkt vermessen oder werden bei -20°C gelagert. Eine genaue Bestimmung der Konzentration (µg/ml GAG im Überstand) von GAG zu gewährleisten müssen sich die Messwerte im linearen Bereich der Standardkurve befinden. Um dies zu gewährleisten werden routinemäßig verschiedene Verdünnungen vorgelegt (1/5, 1/10, 1/20, 1/40). Die Verdünnungen werden mit Medium hergestellt und automatisiert (Hamilton) in einer 384-Multiwellplatte vorgelegt (15 µl). Ebenfalls automatisiert (oder mit Mehrkanalpipette) werden 60 µl DMMB-Lösung zugegeben. Es erfolgt eine schnelle Farbreaktion die anschließend bei 633 nm mit einem Platten-Reader (z.B. Envision) gemessen wird. Je nach vorhandener Probenmenge wird mindestens eine Doppelbestimmung durchgeführt.

Die Daten werden vom MTP-Reader als csv oder xls-Files zur Verfügung gestellt und basierend auf diesem Format (xls) als Rohdaten gespeichert bzw. zur Berechnung des prozentualen Effekts der jeweiligen Verbindung herangezogen.

### 5.) Qualitätskontrollen

Als Kontrolle für die Induktion des pH-induzierten Knorpelabbaus werden 4 Explantate bei pH 7,4 inkubiert. Dies entspricht dem physiologischen pH-Wert des Knorpels und somit ist hier kein Effekt auf die Freisetzung von GAG zu erwarten. Diese GAG Werte (µg/ml Überstand) sind somit immer signifikant niedriger als die GAG-Werte einer Inkukation mit pH 5,5. Eine weitere Kontrolle, die sowohl der Überprüfung des Experimentes dient aber auch wichtig für die Definition des Wirkungsfensters ist, ist die Pepstatin-Kontrolle (pH 5,5 + 0,01 µM Pepstatin A). Diese Substanz blockiert unspezifisch die Aktivität der meisten Proteasen und legt somit den maximal möglichen Effekt einer Verbindung fest.

### 6.) Ergebnisse

Pepstatin zeigt im GAG-Assay einen IC₅₀-Wert im nanomolaren Bereich (siehe Tabelle 1).
(1) Klompmakers, A. & Hendriks, T. (1986) Anal. Biochem. 153, 80-84, Spektrophotometrischer Nachweis für sulfatierte Glycosaminoglycane.
(2) Groves, P.J. et al. (1997) Anal. Biochem. 245, 247-248 Polyvinylalkohol-stabilisierte Bindung von sulfatierten GAGs an Dimethylmethylenblau.

### Beispiel 4: Untersuchung anti-hyperalgetischer Wirkung im Tier

Zur Induktion einer Entzündungsreaktion wurde eine Carrageenan-Lösung (CAR, 1%, 50 µl) einseitig intraartikulär in ein Rattenkniegelenk injiziert. Die nichtinjizierte Seite wurde zu Kontrollzwecken herangezogen. Es wurden sechs Tiere pro Gruppe verwendet. Die Schwellung wurde mittels einer Mikrometerschraube bestimmt (medial-lateral am Kniegelenk) und die thermische Hyperalgesie mittels einer gerichteten Infrarotlichtquelle nach der Hargreaves-Methode (Hargreaves et al. 1988) an der Fußunterseite bestimmt. Da der Ort der Entzündung (Kniegelenk) von dem Ort der Messung (Pfotenunterseite) abweicht, spricht man hier von sekundärer thermischer Hyperalgesie, deren Mechanismen für die Auffindung wirksamer Analgetika von Bedeutung ist.

Versuchsbeschreibung Thermische Hyperalgesie (Hargreaves Test): Das Versuchstier wird in einer Plastikkammer auf eine Quarzglasscheibe gesetzt. Vor der Testung wird dem Versuchstier zunächst etwa 5 - 15 Minuten Zeit gegeben, sich an die Umgebung zu gewöhnen. Sobald sich das Versuchstier nach der Erkundungsphase nicht mehr so oft bewegt (Ende der Explorationsphase), wird die Infrarotlichtquelle, deren Fokus in der Ebene des Glasbodens liegt, direkt unterhalb der zu stimulierenden Hinterpfote positioniert. Ein Versuchsdurchlauf wird nun durch Knopfdruck gestartet: Infrarotlicht führt zur Erhöhung der Hauttemperatur der Hinterpfote. Der Versuchablauf wird entweder bei Anheben der Hinterpfote durch das Versuchstier (als Ausdruck des Erreichens der Schmerzschwelle) oder bei Erreichen einer vorgegeben Höchsttemperatur durch automatisches Ausschalten der Infrarotlichtquelle beendet. Solange das Versuchstier still sitzt, wird Licht, das von der Pfote reflektiert wird, registriert. Ein Wegziehen der Pfote unterbricht diese Reflektion, woraufhin die Infrarotlichtquelle abgeschaltet und die Zeit von An- bis Abschalten registriert wird. Das Gerät ist so geeicht, dass die Infrarotlichtquelle in 10s die Hauttemperatur auf ca. 45 Grad Celsius erhöht (Hargreaves et al. 1988). Zur Testung wird ein von der Firma Ugo Basile zu diesem Zweck produziertes Gerät benutzt.

CAR wurde von Sigma-Aldrich bezogen. Die Applikation der erfimndungsgemäßen, spezifischen Cathepsin D-Inhibitoren wurde 30 Minuten vor der CAR intraartikulär vorgenommen. Als Positivkontrolle wurde Triamcinolone (TAC) 10 µg/Gelenk und als Negativkontrolle wurde das Lösungsmittel (Vehikel) verwendet. Die Hyperalgesie wird als Differenz der Wegziehzeiten zwischen der entzündeten und der nicht-entzündeten Pfote angegeben.

Ergebnis: TAC war in der Lage, die CAR-induzierte Schwellung zu reduzieren, nicht aber Pepstatin. Im Gegensatz dazu konnte Pepstatin das Ausmaß der thermischen Hyperalgesie dosisabhängig reduzieren. Beurteilung: Es konnte gezeigt werden, dass Pepstatin eine antihyperalgetische Wirkung ausübt. Dies kann postuliert werden, da Pepstatin keinen Einfluss auf die entzündliche Schwellung und damit auf den Auslöser der Hyperalgesie zeigt. Es kann damit angenommen werden, dass Pepstatin beim Menschen eine Schmerz reduzierende Wirkung entfaltet.

### Beispiel 5: Stabilität von Pepstatin in boviner Synovialflüssigkeit

### 1.) Gewinnung von boviner Synovialflüssigkeit

Bei der Präparation von bovinen Explantaten (für die Diffusionskammer oder andere Assays) werden entweder Rinderhufe (metacarpale Gelenke) oder Rinderknie verwendet. Die Synovialflüssigkeit lässt sich aus beiden Gelenken gewinnen. Dazu wird bei der Öffnung des Gelenkes die Synovialflüssigkeit mit einer 10 ml Spritze und einer Kanüle vorsichtig aus dem Gelenk entfernt und in bereit gestellte 2 ml Eppendorfgefäße gefüllt. Die Eppendorfgefäße sind je nach Tier beschriftet (Rinderpass liegt vor). Dabei ist darauf zu achten, dass bei der Präparation der Gelenke kein Blut in den Gelenkspalt eindringt. Ist dies der Fall, verfärbt sich die Synovialflüssigkeit rötlich und muss somit verworfen werden. Die Synovialflüssigkeit ist grundsätzlich hochviskos und klar bis gelblich gefärbt. Die Entnahme zusammen mit einer makroskopischen Analyse der Synovialflüssigkeit wird dokumentiert.

### 2.) Ansatz der Stabilitätsprüfung von Substanzen in SF

Um die Stabilität einzelner Verbindungen zu überprüfen wird ein Pool aus 4 verschiedenen bovinen Synovialflüssigkeiten gemischt. Dazu werden ca. 1 ml je SF verwendet. Das Gemisch wird direkt in einem 5 ml Glasgefäß angesetzt um eventuelle Absorptionseffekte zu minimieren. Die SFs werden gründlich aber vorsichtig gemischt. Dabei sollten keine Luftblasen oder Schaum entstehen. Dazu wird ein Vortex-Gerät auf niedrigster Stufe verwendet. Die zu testenden Verbindungen werden in einer Anfangskonzentration (sofern nicht anders angefordert) von 1 µM getestet. Nach Zugabe der Substanz erfolgt eine erneute gründliche und vorsichte Durchmischung des Ansatzes. Zur optischen Kontrolle werden alle SF-Ansätze fotografiert und die Bilder im eLabBio-Ordner des entsprechenden Versuchs abgelegt. Die Ansätze werden für 48 h bei 37°C und 7,5% CO₂ im Brutschrank inkubiert.

### 3.) Probenentnahme

Die Probenentnahme erfolgt nach den vorher abgestimmten Zeiten (sofern nicht anders angefordert, siehe unten). Dabei werden pro Zeitpunkt jeweils 4x 200 µl der SF aus dem Gemisch entnommen und direkt in 0,5 ml "Low-binding" Eppendorf Gefäße überführt. "Low-binding" Eppendorf Gefäße werden verwendet um eine Wechselwirkung der Substanzen mit dem Kunststoff der Gefäße zu minimieren. In dem Eppendorfgefäß wurden bereits jeweils 200 µl Acetonitril vorgelegt, sodass danach eine 1 + 1 Mischung der SF entsteht. Dies erleichtert die folgende Analytik, es kann jedoch direkt nach Zugabe der SF zum Ausfallen vom Protein kommen. Dies ist im Protokoll zu vermerken. Direkt nach Zugabe der Substanz wird die 0 h Probe entnommen. Dies entspricht dem 100% Wert in der Stabilitätsberechnung. Idealerweise sollte sich hier die eingesetzte Konzentration wiederfinden. Die Proben können bei -20°C eingefroren werden.
- 0 h
- 6 h
- 24 h
- 48 h

Als Negativkontrolle wird SF ohne Substanz verwendet. Als Positivkontrolle wird SF mit 1µM Substanz verwendet. Dies entspricht dem 0h Wert und somit 100% Stabilität.

Die Lagerung der Proben erfolgt in "low-Binding" Eppendorf-Gefäßen bei - 20°C. Anschließend werden die Proben quantitativ vermessen. Der Nachweis der entsprechenden Substanzen erfolgt massenspektrometrisch.

### 4.) Datenverarbeitung

Die gemessenen Konzentrationen (ng/ml) werden in einer Graphik (GraphPad Prism®) als zeitlicher Verlauf dargestellt. Dabei wird die prozentuale Stabilität der Substanz ermittelt. Als 100% Wert wird der Ausgangswert in SF zum Zeitpunkt 0 h verwendet. Die Daten werden unter der jeweiligen Versuchsnummer in eLabBio gespeichert und in die MSR Datenbank (als Prozent Stabilität nach den entsprechenden Inkubationszeiten) berichtet.

### 5.) Ergebnisse

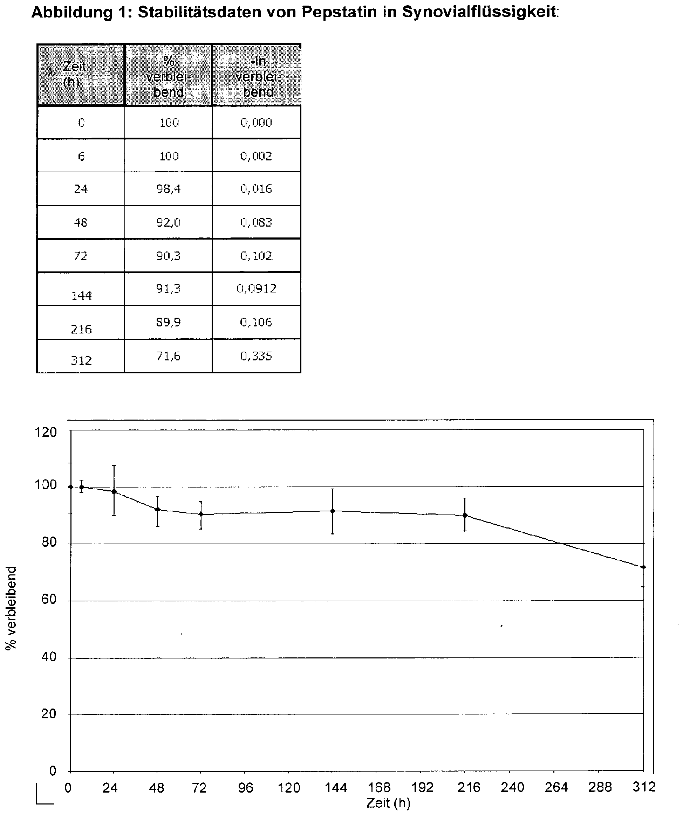

Pepstatin bleibt über einen Zeitraum von wenigstens zwei Wochen in Synovialflüsigkeit stabil (siehe Abbildung 1).

### Beispiel 6: Pharmakokinetische Daten nach intraartikulärer Injektion

Für diese Studie (KK-Rat-12-003) wurden 14 männliche Lister Hooded Ratten verwendet. Alle Ratten erhielten einmalig eine intraartikuläre Injektion in beide Kniegelenke zum Zeitpunkt "0". Eine Injektion bestand aus 30 µl, in welcher ca. 700 µg Coumpound homogen suspendiert wurden (Suspension = Coumpound in 0,5% Methocel K4M mit 0,25% Tween20 in PBS). Die Suspension wurde via einer 25G-Kanüle appliziert. Besonderheiten bezüglich etwaiger Veränderungen von in-life-Parametern wie z.B. Körpergewicht, Knieschwellungen oder Schonhaltungen wurden nicht verzeichnet. Zu jedem angegebenen Zeitpunkt in Tabelle 2 wurden Tiere getötet, die Kniegelenke präpariert (Entfernung von Haut- und Muskelgewebe) und zur weiteren Aufarbeitung eingefroren.

Die tiefgefrorenen Gelenke wurden kurz angetaut, mit der Knochenschere so gut wie möglich zerkleinert und anschließend das 4-fache Volumen an 80%igem Ethanol zugegeben. Anschließend wurde mit dem Ultraturrax homogenisiert, der Extrakt wurde 20 min bei RT geschüttelt und dann mindestens 30 min bei -20°C gelagert. Danach wurde 5 min bei 13000 rpm zentrifugiert, ein 10 µl-Atiquot des Überstandes 1:5000 mit interner Standardlösung verdünnt, in eine PCR-Plate überführt und analysiert.

20 µl Plasma wurde mit 20 µl interner Standardlösung versetzt, 100 µl Methanol zugegeben und 5 min geschüttelt. Danach wurden die Extrakte mindestens 30 min bei -20°C gelagert und anschließend 5 min bei 13000 rpm zentrifugiert. 80 µl des Überstandes wurden in eine PCR-Plate überführt und analysiert.

Alle Proben wurden mit Hilfe eines UPLC-MS/MS Systems analysiert. Die Nachweisgrenzen für Pepstatin waren 0.1 ng/ml in Plasma und 8 µg/g im Gewebe.

**Tabelle 2 - Kniegelenksdurchmesser in mm**

| Tage nach Injektion | Mittelwert Gelenksdurchmesser [mm] | SD | N (injected joints) |
|---|---|---|---|
| 0 | 10,08 | 0,31746745 | 28 |
| 1 | 10,88 | 0,49042038 | 26 |
| 2 | 10,50 | 0,30862869 | 16 |
| 4 | 10,12 | 0,19734488 | 16 |
| 7 | 10,35 | 0,19478086 | 16 |
| 9 | 10,14 | 0,18101258 | 10 |
| 11 | 10,37 | 0,15579188 | 10 |
| 13 | 10,24 | 0,17707444 | 8 |
| 15 | 10,45 | 0,15238579 | 8 |
| 20 | 10,18 | 0,15942605 | 6 |
| 27 | 10,50 | 0,11189281 | 6 |

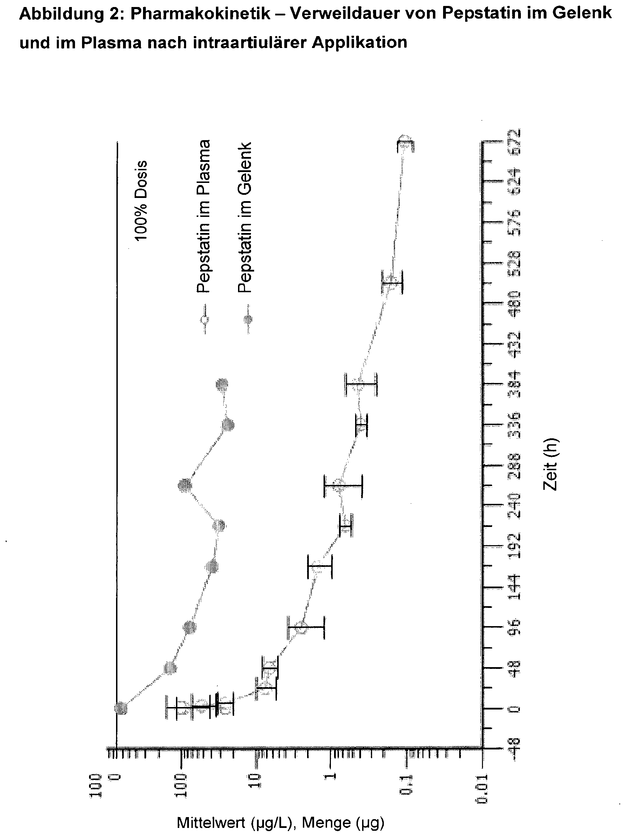

Nach intra-artikulärer Gabe einer Suspension zeigt Pepstatin eine mittlere Verweilzeit von ca. 106 h im Kniegelenk in der Ratte und aufgrund der langsamen Freisetzung aus dem Synovium und sehr hohen Clearance im Blut nur eine sehr niedrige systemische Exposition. Im Plasma zeigt Pepstatin eine sogenannte Flip-Flop Kinetik, d.h. die terminale Plasmahalbwertszeit wird nicht durch die Elimination, sondern durch die Freisetzung von Pepstatin aus der Suspension und Diffussion über sdie Synovialmembran bestimmt. Pepstatin konnte im Plasma bei allerdings sehr niedriger Konzentration bis zu 28 Tage nach Verabreichung nachgewiesen werden (siehe Abbildung 2).

### Beispiel 7: Pharmakokinetische Daten nach intravenöser (i.v.) und oraler (p.o.) Verabreichung

Die pharmakokinetischen Parameter von Pepstatin wurden in Wistar-Ratten (BW ca. 250 g), nach Verabreichung der Testsubstanz in einem Cocktail von bis zu 4 Substanzen, bestimmt. Pepstatin wurde den männlichen Ratten (n=3 pro Verabreichungsart) entweder mittles einer i.v. Bolusinjektion in die Schwanzvene oder über eine orale Gavage mittles einer Edelstahlkanüle verabreicht. Die Testsubstanzen wurden in DMSO/PEG200/Wasser (2/60/38 v/v) mit einer finale Konzentration von 0.8 mg/mL gelöst und eine Dosis von 0.2 mg/kg i.v. und 0.5 mg/kg oral verabreicht. Blutproben (200 µl) wurden unter einer leichten Isofluran-Narkose über die sublinguale Vene zu den folgenden Zeitpunkten nach Verabreichung genommen: iv: 0.1, 0.5, 1, 2, 4, 6 und 24 h; po: 0.25, 0.5, 1, 2, 4, 6 und 24 h.

Die Blutproben wurden in Li-Heparin-haltigen Zentrifugenröhrchen gesammelt und 3 min bei ca. 10000 g bei 4°C zentrifugiert. Das daraus erhaltene Plasma wurde sofort bei -20°C eingefroren und bis zur Analyse aufbewahrt. Die Plasmakonzentrationen wurden mittels einer standard LC-MS/MS Methode bestimmt. Die pharmakokinetischen Parameter (Clp, Vss, T1/2, F) wurden über eine NCA Analyse ermittelt.

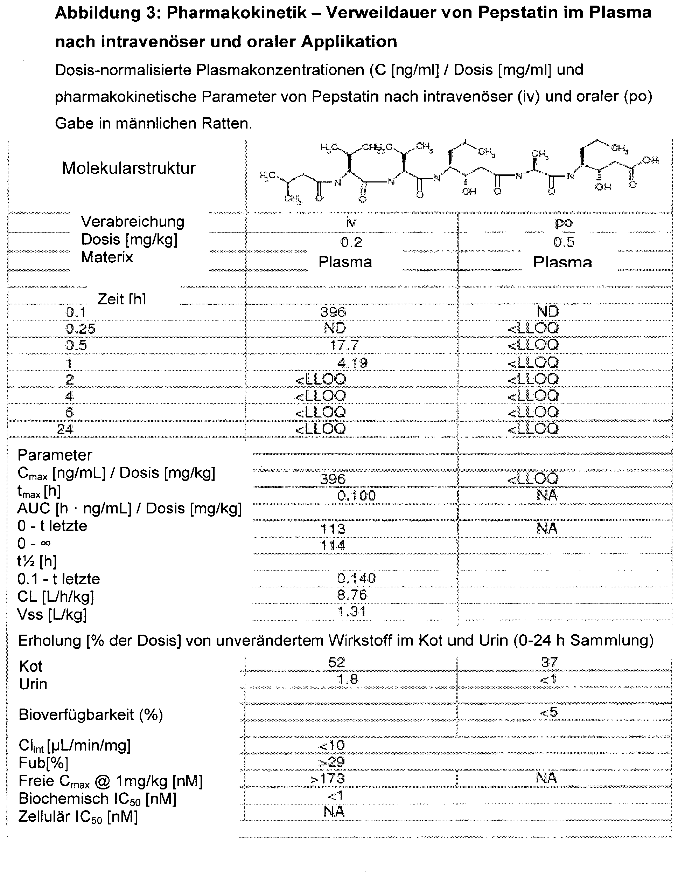

Nach intravenöser Gabe zeigt Pepstatin eine sehr hohe Clearance (> 100% Leberblutfluß), ein mittleres Verteilungsvolumen und darausfolgend eine sehr kurze Plasmahalbwertszeit (ca. 0.14 h). Nach oraler Gabe befanden sich alle Plasmakonzentrationen unterhalb der Nachweisgrenze (siehe Abbildung 3).

### Beispiel 8: Wirksamkeit (in vivo) von Pepstatin bei Arthrose - ACLT tMx Modell

Als Versuchstiere wurden Ratten gewählt. Nach der Rasur und Desinfektion wird mittels etwa 1 cm langem medialen Hautschnitts das Operationsgebiet eröffnet. Die Kniegelenkskapsel wird präpariert und das mediale Patellarband dargestellt. Nach Eröffnen der Gelenkkapsel, Durchtrennen der medialen parapatellaren Bänder und Verlagerung der Patella nach lateral wird das vordere Kreuzband mit einem gebogenen, stumpf endenden Messer durchtrennt (ACLT = Anterior Cruciate Ligament Transection). Anschließend werden das vordere und hintere den Meniskus fixierende Band präpariert und durchtrennt und der Meniskus entnommen tMx (Resektomie des medialen Meniskus). Nach Repositionierung der Patella wird das Gelenk abschließend mit steriler Kochsalzlösung gespült um eventuell entstandene Blutkoagel zu entfernen.

Die Patella wird repositioniert und das mediale Kniescheibenband mittels fortlaufender Naht unter Verschluss der Kapsel wieder fixiert. Die Muskulatur wird ebenfalls vernäht. Mittels Einzelheften wird anschließend die Haut verschlossen. Die Operationsdauer beträgt etwa 10 Minuten. Die postoperative Versuchsdauer betrug 6 Wochen.

Zur intraartikulären Injektion werden die Tiere mit 1,5-2 Vol% Isofluran narkotisiert. Vor der Injektion wird Buprenorphin subkutan injiziert. Das Injektionsgebiet wird schonend rasiert und desinfiziert. Das Kniegelenk wird in eine leicht gebeugte Stellung gebracht und die zu testende Substanz oder das Vehikel werden in das Gelenk injiziert.

Für den vorliegenden Versuch wurde 1mg Pepstatin in 30µl eingesetzt. Als negative Kontrolle wurde einer Versuchsgruppe nur Vehikel injiziert. Als positive Kontrolle wurde jeweils das nicht operierte Hinterbein der Ratte verwendet.

### Prozessierung in der Histologie

Die entnommenen Gewebproben werden über mindestens 72 Stunden in Paraformaledhyd (4%) fixiert und danach 24 Stunden unter fließendem Leitungswasser gespült. Die Proben werden anschließend mittels Osteosoft über einen Zeitraum von 4 Wochen entkalkt. Darauf folgten die Infiltration des Gewebes mit Paraffin und die Anfertigung histologischer Schnitte mit 7 µm Dicke. Zur Beurteilung wurde die Schnitte mit Safranin O Fast Green gefärbt.

### Bewertung

Von jedem Tier wurden zwei Schnitte im Bereich der Belastungszone ausgewählt und von zwei erfahrenen Personen anhand eines Berwertungssystems beurteilt. Das Bewertungssystem basiert auf der Arbeit von V.B. Kraus et al (Osteoarthritis & Cartilage, 18, S3, 2010).

### Ergebnisse

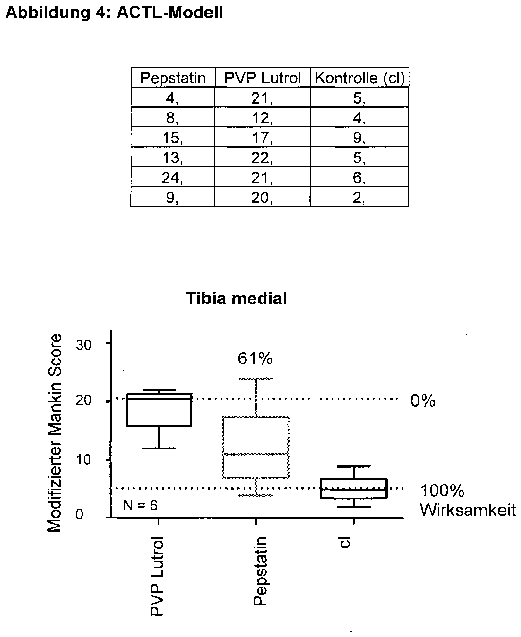

Pepstatin zeigt auch *in vivo* eine signifikante Wirksamkeit bei Arthrose (siehe Abbildung 4)

### Beispiel 9: Mikrodialyse

Für die Wirkung eines Arzneimittels sind die freien Arzneimittelspiegel am Wirkort entscheidend. Bei intraartikulären vorgenommenen Injektionen ist der Verteilungsraum in der Synovialflüssigkeit diesbezüglich von großem Interesse.

Da die Synovialflüssigkeit einerseits eine komplexe Matrix für die analytische Bestimmung von Peptiden darstellt und andererseits nur die freien, d.h. der nicht proteingebundene Arzneimittelanteil für die Wirkung relevant ist, wurde das Verfahren der Mikrodialyse eingesetzt. Das gewonnene Eluat aus der Mikrodialyse ermöglicht eine Analytik von freien Arzneimittelspiegeln aus der komplexen Matrix der Synovialflüssigkeit. Grund dafür ist, daß eine Sperrmembran (Mikrodialysemembran) eingesetzt wird, die nur Moleküle bis zu einer bestimmten Größe durchlassen kann. Diese "Porengröße" bestimmt neben der Flußgeschwindigkeit des Eluates und der vorhandenen freien Konzentration des Arzneimittels in der Synovialflüssigkeit als treibende Kraft für den Stoffaustausch die Konzentration des Arzneimittels im Eluat.

Für die hier vorliegenden Untersuchungen wurden Mikrodialysesonden der Firma CMA (Referenznummer 000082; CMA 7; Microdialysis Probe 1 mm 3/pkg) bestückt mit einer Cuprophane Membran und einer Porengröße von 6 kDa verwendet. Der Eluatflow betrug 0.5 µl/min.

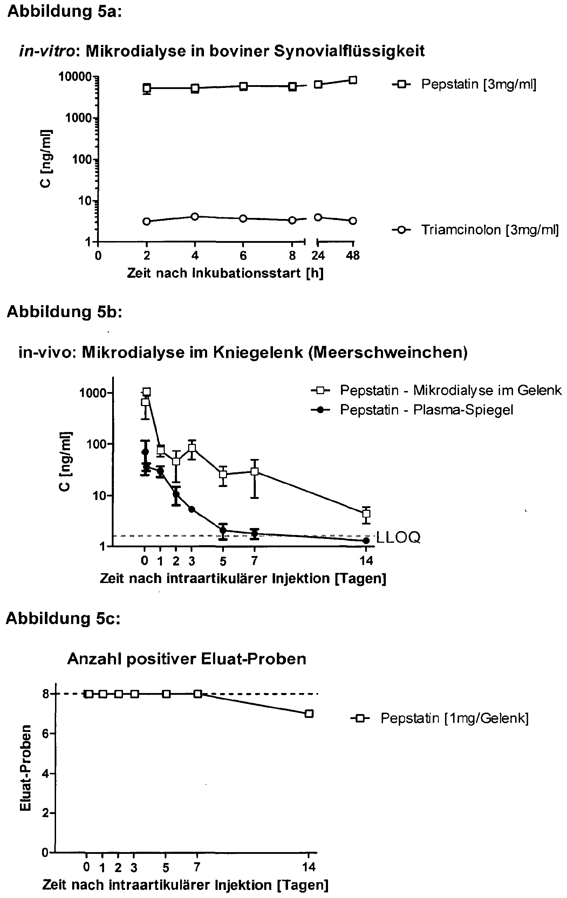

Zunächst wurde eine Freisetzungskinetik in boviner Synovialflüssigkeit untersucht und mit Triamcinolon (Triam Injekt® 20 mg) - ein für die intraartikuläre Injektion zugelassenes Medikament - verglichen. Triamcinolone wurde hier in der bereits gelösten Kristallsuspension eingesetzt wohingegen die Pepstatin-Kristalle direkt und nicht angelöst zur Synovialflüssigkeit dazugegeben wurden. Es wurden 3 mg Arzneimittel pro ml Synovialflüssigkeit in einem Glasgefäß eingesetzt, was in etwa den Konzentrationsverhältnissen nach einer intraartikulären Injektion in das Kniegelenk entspricht. Erstaunlicherweise und in Kontrast zu Triamcinolon wurden sehr hohe Spiegel an ungebundenen Pepstatin gemessen. Auch die mehr oder weniger gleichbleibenden Spiegel sprechen für eine schnelle Auflösungsgeschwindigkeit bei hoher Stabilität von Pepstatin in Synovialflüssigkeit (siehe Abbildung 5a)

Wegen der hohen Löslichkeit von Pepstatin in Synovialflüssigkeit sind die Ergebnisse aus der Gelenksmikrodialyse am Meerschweinchen (Dunkin Hartley) überraschend: Bis zu 14 Tage nach einer intraartikulären Injektion von Pepstatin [1 mg/Gelenk appliziert in 50 µl als Suspension] sind signifikante Spiegel von Pepstatin im Gelenk nachweisbar und das in fast allen untersuchten Eluaten (siehe Abbildungen 5b und 5c).

## Patentansprüche

1. Pharmazeutische Zubereitung enthaltend Pepstatin und/oder eines seiner physiologisch unbedenklichen Salze, Solvate und Stereoisomere, einschließlich deren Mischungen in allen Verhältnissen und weitere Träger- und/oder Hilfsstoffe, zur Verwendung als Medikament bei der Behandlung und/oder Prophylaxe von physiologischen und/oder pathophysiologischen Zuständen, ausgewählt aus der Gruppe bestehend aus Arthrose, traumatischen Knorpelverletzungen, Arthritis, Schmerz, Allodynie oder Hyperalgesie, wobei die pharmazeutische Zubereitung wie nachfolgend intraartikulär appliziert wird:
a) wöchentlich bis jährlich,
b) 14-tägig bis halbjährlich oder
c) monatlich bis vierteljährlich.

2. Pharmazeutische Zubereitung zur Verwendung nach Anspruch 1, wobei die pharmazeutische Zubereitung wenigstens einen weiteren Arzneimittelwirkstoff enthält.

3. Pharmazeutische Zubereitung zur Verwendung nach Anspruch 1 oder 2, zur Verwendung als Medikament bei der Behandlung und/oder Prophylaxe von Arthrose.

4. Pepstatin und/oder eines seiner physiologisch unbedenklichen Salze, Solvate und Stereoisomere, einschließlich deren Mischungen in allen Verhältnissen zur Verwendung als Medikament bei der Behandlung und/oder Prophylaxe von physiologischen und/oder pathophysiologischen Zuständen, ausgewählt aus der Gruppe bestehend aus Arthrose, traumatische Knorpelverletzungen, Arthritis, Schmerz, Allodynie und Hyperalgesie, wobei Pepstatin und/oder eines seiner physiologisch unbedenklichen Salze, Solvate und Stereoisomere, einschließlich deren Mischungen in allen Verhältnissen wie nachfolgend intraartikulär appliziert wird:
a) wöchentlich bis jährlich,
b) 14-tägig bis halbjährlich oder
c) monatlich bis vierteljährlich.

5. Pepstatin und/oder eines seiner physiologisch unbedenklichen Salze, Solvate und Stereoisomere, einschließlich deren Mischungen in allen Verhältnissen nach Anspruch 4 zur Verwendung als Medikament bei der Behandlung und/oder Prophylaxe von Arthrose.

## Claims

1. Pharmaceutical preparation comprising pepstatin and/or one of its physiologically acceptable salts, solvates and stereoisomers, including mixtures thereof in all ratios, and further excipients and/or adjuvants, for use as medicament in the treatment and/or prophylaxis of physiological and/or pathophysiological conditions, selected from the group consisting of osteoarthritis, traumatic cartilage injuries, arthritis, pain, allodynia or hyperalgesia, where the pharmaceutical preparation is administered intra-articularly as follows:
a) weekly to yearly,
b) fortnightly to half-yearly or
c) monthly to quarter-yearly.

2. Pharmaceutical preparation for use according to Claim 1, where the pharmaceutical preparation comprises at least one further medicament active compound.

3. Pharmaceutical preparation for use according to Claim 1 or 2, for use as medicament in the treatment and/or prophylaxis of osteoarthritis.

4. Pepstatin and/or one of its physiologically acceptable salts, solvates and stereoisomers, including mixtures thereof in all ratios, for use as medicament in the treatment and/or prophylaxis of physiological and/or pathophysiological conditions, selected from the group consisting of osteoarthritis, traumatic cartilage injuries, arthritis, pain, allodynia or hyperalgesia, where pepstatin and/or one of its physiologically acceptable salts, solvates and stereoisomers, including mixtures thereof in all ratios, is administered intra-articularly as follows:
a) weekly to yearly,
b) fortnightly to half-yearly or
c) monthly to quarter-yearly.

5. Pepstatin and/or one of its physiologically acceptable salts, solvates and stereoisomers, including mixtures thereof in all ratios according to Claim 4, for use as medicament in the treatment and/or prophylaxis of osteoarthritis.

## Revendications

1. Préparation pharmaceutique comprenant de la pepstatine et/ou l'un de ses sels, solvates et stéréoisomères physiologiquement acceptables, y compris des mélanges de ceux-ci en toutes proportions, et en outre des excipients et/ou des adjuvants, pour une utilisation comme médicament destiné au traitement et/ou à la prophylaxie de conditions physiologiques et/ou physiopathologiques, choisies dans le groupe constitué par l'ostéo-arthrite, les lésions traumatiques du cartilage, l'arthrite, la douleur, l'allodynie ou l'hyperalgésie, où la préparation pharmaceutique est administrée par voie intra-articulaire comme suit :
a) d'une fois par semaine à une fois par an,
b) d'une fois tous les 15 jours à une fois par semestre ou
c) d'une fois par mois à une fois par trimestre.

2. Préparation pharmaceutique pour une utilisation selon la revendication 1, où la préparation pharmaceutique comprend au moins un autre composé actif médicamenteux.

3. Préparation pharmaceutique pour une utilisation selon la revendication 1 ou 2, pour une utilisation comme médicament destiné au traitement et/ou à la prophylaxie de l'ostéo-arthrite.

4. Pepstatine et/ou l'un de ses sels, solvates et stéréoisomères physiologiquement acceptables, y compris des mélanges de ceux-ci en toutes proportions, pour une utilisation comme médicament destiné au traitement et/ou à la prophylaxie de conditions physiologiques et/ou physiopathologiques, choisies dans le groupe constitué par l'ostéo-arthrite, les lésions traumatiques du cartilage, l'arthrite, la douleur, l'allodynie ou l'hyper-algésie, où la pepstatine et/ou l'un de ses sels, solvates et stéréoisomères physiologiquement acceptables, y compris des mélanges de ceux-ci en toutes proportions, est administrée par voie intra-articulaire comme suit :
a) d'une fois par semaine à une fois par an,
b) d'une fois tous les 15 jours à une fois par semestre ou
c) d'une fois par mois à une fois par trimestre.

5. Pepstatine et/ou l'un de ses sels, solvates et stéréoisomères physiologiquement acceptables, y compris des mélanges de ceux-ci en toutes proportions selon la revendication 4, pour une utilisation comme médicament destiné au traitement et/ou à la prophylaxie de l'ostéo-arthrite.
